(19) Europäisches Patentamt / European Patent Office / Office européen des brevets

(11) **EP 4 595 944 A1**

(12) **EUROPEAN PATENT APPLICATION**
published in accordance with Art. 153(4) EPC

(43) Date of publication:
06.08.2025 Bulletin 2025/32

(21) Application number: 23871502.3

(22) Date of filing: 09.08.2023

(51) International Patent Classification (IPC):
*A61K 6/849* (2020.01)    *A61C 5/60* (2017.01)
*A61C 13/00* (2006.01)    *A61C 13/15* (2006.01)
*A61K 6/878* (2020.01)    *A61K 6/887* (2020.01)
*C01B 33/18* (2006.01)

(52) Cooperative Patent Classification (CPC):
A61C 5/60; A61C 13/00; A61C 19/003;
A61K 6/849; A61K 6/878; A61K 6/887; C01B 33/18

(86) International application number:
PCT/JP2023/029044

(87) International publication number:
WO 2024/070260 (04.04.2024 Gazette 2024/14)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 27.09.2022 JP 2022153435

(71) Applicant: Tokuyama Dental Corporation
Tokyo 110-0016 (JP)

(72) Inventors:
• **OYA, Naoyuki**
  **Tokyo 110-0016 (JP)**
• **MORISAKI, Hiroshi**
  **Tokyo 110-0016 (JP)**

(74) Representative: **Lorenz & Kollegen**
**Lorenz & Kollegen Patentanwälte**
**Partnerschaftgesellschaft mbB**
**Alte Ulmer Straße 2**
**89522 Heidenheim (DE)**

(54) **DENTAL CURABLE COMPOSITION, BLANK FOR DENTAL-CUTTING WORK, PRODUCTION METHOD FOR SILICA-BASED COMPOSITE OXIDE POWDER BODY, AND DENTAL FILLER**

(57)    [Object] To provide a dental blank for cutting, which includes a to-be-cut portion formed of a hybrid resin that includes a spherical particle of a silica composite oxide and has an external color unlikely to change depending on a thickness.

[Solving Means] When a raw material solution in which a condensable compound such as tetraethyl silicate and tetra(n-butoxy)zirconate is dissolved is added to a basic solution to precipitate a silica composite oxide particulate of a silica composite oxide, a cured body of a dental curable composition that includes 100 to 800 parts by mass of a silica composite oxide particulate with respect to 100 parts by mass of a radically polymerizable monomer is used as a to-be-cut portion, the silica composite oxide particulate being obtained by controlling a solvent composition of each of the solutions, including a spherical particle of a silica composite oxide, and having an average primary particle size of 350 to 600 nm, the spherical particle having a silica content ratio of 80 to 92 mol% and including no silica core.

EP 4 595 944 A1

**Description**

Technical Field

The present invention relates to a dental curable composition, a dental blank for cutting, a silica composite oxide particulate, and a dental filler.

Background Art

A composite resin used in dental filling treatment (hereinafter, abbreviated as "CR" in some cases) is a dental curable composition that provides a cured body called a hybrid resin (hereinafter, abbreviated as "HR" in some cases). Further, the HR is also widely used as a material for inlays, onlays, crowns, bridges, implant superstructures, and the like used in dental prosthetic restorations.

The dental curable composition that is a raw material of the HR is often formed of a composition including an inorganic filler such as silica, a polymerizable monomer such as a methacrylate resin, and a polymerization initiator from the viewpoint of strength, etc. As the inorganic filler, a particulate (inorganic particulate) formed of a spherical inorganic oxide particle is often used from the viewpoint of workability in cutting and polishing and high aesthetics (see Patent Literature 1). Then, as the filler that is the particulate of a spherical inorganic oxide particle, a particulate formed of a spherical particle of a silica composite oxide that is an inorganic oxide including, as main components, at least one metal component selected from the group consisting of Groups I to IV of the periodic table and a silicon component, particularly, a silica-Group 4 composite oxide such as a silica-zirconia oxide is generally used (see Patent Literatures 1 to 3).

Note that, in general, it is said that the particulate formed of a spherical particle of a silica composite oxide is produced by a so-called sol-gel method in which a hydrolysable (condensable) compound such as alkoxides of silicon and various metals undergo hydrolysis and dehydration condensation in a basic aqueous solution, and in the above sol-gel method, when a core (seed or nucleus for grain growth) of silica is formed by adding only a raw material compound of silica to the basic aqueous solution and then a mixture of a raw material compound of silica and a raw material compound of a metal oxide is added to grow a composite oxide layer around the core of silica, the particle size distribution of the obtained particulate can be made favorable (particulate formed of spherical particles with a uniform particle diameter can be obtained) (Patent Literature 3). Then, the particulate formed of a spherical particle of a silica-Group 4 composite oxide, which is used as a dental filler, is often produced by such a method (see, for example, Production Examples 5 and 6 of Patent Literature 2).

As a dental blank for cutting, which has become increasingly popular in recent years with the advancement of digital technologies, a dental hybrid resin blank for cutting (hereinafter, the dental hybrid resin blank for cutting will be also referred to simply as "HR blank") including a to-be-cut portion formed of an HR in which the particulate of a silica-Group 4 composite oxide described above is blended has been known (see Patent Literature 4). Note that the dental blank for cutting means a dedicated workpiece (also called a mill blank) that can be attached to a cutting machine of a cutting system (CAD/CAM system) using a computer aided design (CAD) and computer aided manufacturing (CAM) technology, and usually include a block-shaped to-be-cut portion that is formed of a non-metal material and has a predetermined shape and a member for attaching this to the cutting machine. Then, using the CAD/CAM system, the to-be-cut portion is cut (CAM) into the prosthetic shape designed by CAD on the basis of digital information such as the shape of the oral cavity and the shape of the model, thereby preparing a dental restoration having a desired shape with high accuracy.

Incidentally, in the case where the HR is used as a dental prosthetic material, it is necessary to use one that has been toned to a color close to the color (expressed as an index including a combination of hue and a mixed index of brightness and saturation, or an index taking into account hue, brightness, and saturation. Hereinafter, the color specified by such an index will referred to also as "shade") of natural teeth using a coloring agent such as a pigment from the viewpoint of aesthetics. Since the color (shade) of natural teeth varies from person to person, it is common to prepare HR and CR products toned to a plurality of different predetermined colors (shades) and select and use one that matches the color of the tooth to be restored or the color (shade) of the surrounding teeth.

Such color (shade) selection (generally, referred to as "shade taking") is generally made using teeth color samples called a shade guide. There are various shade guides with the number of color samples and the configuration of a holding device that holds the color samples that are designed to make the color judgement work easier, and "VITA Classical" (product name) manufactured by VITA, which includes a total of 16 color samples and allows the color of a restoration portion to be determined by comparing the shade guide with the colors of the restoration portion and the surrounding teeth, is most widely used. In the VITA shade guide, the colors in the groups A to D are classified by brightness and are represented by symbols. That is, the colors classified into the group A (reddish brown), the group B (reddish yellow), the group C (greyish), and the group D (reddish-grey), and the 16 shades are arranged in order of brightness (high -> low) as follows: "B1 -> A1 -> B2 -> D2 -> A2 -> C1 -> C2 -> D4 -> A3 -> D3 -> B3 -> A3.5 -> B4 -> C3 -> A4 -> C4". When HR dental materials and CR are commercialized, those in which the shades (of external colors of the HR or CR cured bodies) are the above 16 shades or some shades selected therefrom are often available.

Citation List

Patent Literature

Patent Literature 1: Japanese Examined Patent Publication No. 1991-10603
Patent Literature 2: Japanese Patent Application Laid-open No. 1996-12305
Patent Literature 3: Japanese Examined Patent Publication No. 1989-38044
Patent Literature 4: Japanese Patent Application Laid-open No. 2017-213394

Disclosure of Invention

Technical Problem

As a result of the present inventors' investigations, it was confirmed that the HR blended as an inorganic composition as described in Patent Literature 2 using a silica composite oxide particulate of a silica-zirconia oxide synthesized using a sol-gel method as described in Patent Literature 2 provides excellent mechanical strength, wear resistance, and surface smoothness. Meanwhile, it was found that the color tone of the toned HR observed visually varies depending on the thickness in some cases. Specifically, it was found that the blue color becomes stronger in the case where the thickness of the HR is thin in the form of a tooth crown and the red color becomes stronger in the case where the thickness is thick. Note that it is well known that the HR has a certain degree of transparency and therefore the shade of the external color changes depending on the thickness. However, it is hardly recognized that the balance between the blue color and the red color changes depending on the thickness.

If the color tone changes depending on the thickness of the HR, it can cause confusion in shade taking of an HR dental material or CR provided as a product depending on the degree of the change. That is, the shades available for each product need to be determined by the external color of the HR (or CR cured body) having a predetermined thickness (hereinafter, referred to also as "reference thickness"). If there is a difference between the thickness of the HR (or CR cured body) in the actual usage form (processed into a prosthesis or deposited) and the reference thickness, a color shift (degree of discrepancy in the external color) occurs in accordance with the magnitude of the difference.

For example, if the shade of an HR blank including a to-be-cut portion that uses an HR of a type that causes the above color shift and has a rectangular parallelepiped shape of a size of approximately 1.5 cm $\times$ 1.5 cm $\times$ 2 cm is determined with the reference thickness of 1 mm, the external color of the to-be-cut portion has the red color stronger than the determined shade. For this reason, not only users who are unaware of the above color shift may mistakenly believe that the shade of the product is incorrect, but also the desired color harmony cannot be obtained in principle when the thickness of the main part of the actually created prosthesis is different from the reference thickness.

In this regard, the present invention aims to clarify in what type of HR the balance between the blue color and the red color in the external color changes depending on the thickness, provide a dental curable composition capable of providing the toned HR that does not cause the above color shift depending on the thickness, and provide an HR blank including a to-be-cut portion formed of the toned HR that does not cause the color shift depending on the thickness.

Solution to Problem

The present invention solves the above problems, and a first embodiment of the present invention is a dental curable composition, including: a radically polymerizable monomer: 100 parts by mass; and a silica composite oxide particulate that includes a composite oxide of silicon and a metal including at least titanium or zirconium, has a silica content ratio of 80 to 92 mol% and an average primary particle size of 350 to 600 nm, and is formed of a spherical particle including no silica core inside: 100 to 800 parts by mass.

In the dental curable composition according the above embodiment (hereinafter, referred to also as the "dental curable composition according to the present invention"), a standard deviation value of the average primary particle size is favorably 1.00 to 1.30.

The dental curable composition according to the present invention favorably further include a dye.

A second embodiment of the present invention is a dental blank for cutting including a to-be-cut portion, characterized in that the to-be-cut portion includes a cured body of a dental curable composition (hereinafter, referred to also as the "dental blank for cutting according to the present invention").

A third embodiment of the present invention is a method of producing a silica composite oxide particulate, including:

a step of preparing a raw material solution by dissolving a condensable silicon compound and a condensable metal compound in a first solvent including a first organic solvent; and

a step of precipitating, by adding the raw material solution to a basic solution including a second solvent that includes a second organic solvent and water to hydrolyze and condense the condensable silicon compound and the condensable metal compound, a silica composite oxide particulate that includes a composite oxide of silicon and a metal including at least titanium or zirconium, has a silica content ratio of 80 to 92 mol%, and is formed of a spherical particle, the method of producing a silica composite oxide particulate being characterized in that

in the first organic solvent, a content ratio of methanol is 80 mass% or more,

in the second organic solvent, a total content ratio of an alcohol having an alkyl group that has 3 carbon atoms and an alcohol having an alkyl group that has 4 to 5 carbon atoms is 80 mass% or more and a content ratio of an alcohol having an alkyl group that has 3 or less carbon atoms is 5 to 50 mass%, and

the silica composite oxide particulate that has an average primary particle size of 350 to 600 nm and includes no silica core inside the spherical particle is precipitated by adding the raw material solution to the basic solution such that a content ratio of an alcohol having an alkyl group that has 3 to 5 carbon atoms is 65 mass% or more with respect to a total mass of the first organic solvent and the second organic solvent.

In the production method according to the above embodiment (hereinafter, referred to also as the "production method according to the present invention"), the step of preparing a raw material solution favorably includes

a step of preparing, by mixing a silicon alkoxide, methanol, water, and an acid, a first composition in which a partial hydrolysate of the silicon alkoxide and/or an oligomer obtained by condensing the partial hydrolysate is dissolved,

a step of preparing, by mixing a metal alkoxide of the metal and a polar organic solvent for dissolving the metal alkoxide, a second composition in which at least one of the metal alkoxide, a partial hydrolysate of the metal alkoxide, or an oligomer obtained by condensing the partial hydrolysate is dissolved, and

a step of preparing the raw material solution by mixing the first composition, and the second composition.

Further, a standard deviation value of the average primary particle size is favorably within a range of 1.00 to 1.30.

A fourth embodiment of the present invention is a dental filler, which is blended in a dental curable composition that includes a radically polymerizable monomer, characterized in that

the dental filler includes a composite oxide of silicon and a metal including at least titanium or zirconium, has a silica content ratio of 80 to 92 mol% and an average primary particle size of 350 to 600 nm, and is a silica composite oxide particulate formed of a spherical particle including no silica core inside, a standard deviation value of the average primary particle size being with a range of 1.00 to 1.30.

In the dental filler according to the above embodiment (hereinafter, referred to also as the "dental filler according to the present invention")

the radically polymerizable monomer is favorably a (meth)acrylic compound radically polymerizable monomer, and

the dental filler favorably satisfies the following formula: $0.01 < n_F - n_P < 0.1$, wherein

$n_F$ represents a refractive index of the spherical particle at 25°C and with respect to light of a wavelength of 589 nm and

$n_P$ represents a refractive index of a cured body of the radically polymerizable monomer at 25°C and with respect to light of a wavelength of 589 nm.

Advantageous Effects of Invention

The dental curable composition according to the present invention not only is capable of providing a cured body having favorable strength, workability in cutting and polishing, and aesthetics as in the existing dental curable composition using a silica composite oxide particulate as an inorganic filler but also has the advantage that the color shift caused by the thickness of the cured body is unlikely to occur by using a silica composite oxide particulate (that also forms the dental filler according to the present invention) that satisfies a specific condition as the particulate.

For this reason, the dental blank for cutting according to the present invention including a to-be-cut portion that includes a cured body of the dental curable composition according to the present invention has the advantage that the product is unlikely to cause an unpleasant feeling because shade taking can be performed on the basis of the external color tone (shade) of the to-be-cut portion despite that a silica composite oxide spherical particle is blended in the to-be-cut portion.

Further, in accordance with the production method according to the present invention, it is possible to efficiently produce a silica composite oxide particulate that is unlikely to cause the color shift due to the thickness of the cured body when being blended in a dental curable composition.

Mode(s) for Carrying Out the Invention

1. Overview of present invention

The present inventors have investigated the cause of the phenomenon in which the external color of the HR differs depending on the thickness in the case where the silica composite oxide particulate of a silica-zirconia oxide synthesized using the sol-gel method as described in Patent Literature 2 is used for the HR. As a result, they have discovered that the above phenomenon is evident in the case where the spherical particle forming the silica composite oxide particulate of a silica-zirconia oxide includes a core (silica core) formed of only a silica component but is unlikely to occur in the case where the spherical particle includes a particle entirely formed of a composite oxide without such a silica core (referred to also as a coreless particle), and it is very difficult to obtain, even if a spherical particle of a silica composite oxide of a silica-Group 4 type such as a silica-zirconia oxide is tried to be produced under the condition that the composition ratio of the Group 4 oxide is high such that no silica core is formed, a particulate formed of a spherical particle having a relatively large particle size, e.g., an average primary particle size of 350 nm or more.

In this regard, the conditions for producing a particulate formed of a spherical coreless particle of a composite oxide of silica and a metal oxide, such as a silica-Group 4 composite oxide, which has an average primary particle size of 350 nm or more, were further investigated. As a result, it has been found that when a raw material solution in which a condensable silicon compound that is a raw material of a silica component and a condensable metal compound that is a raw material of a metal oxide component are dissolved in an organic solvent (first organic solvent) is added to a basic aqueous solution including ammonia water and a water-soluble organic solvent (second organic solvent) to precipitate a spherical particulate, the above-mentioned object can be achieved in the case where (i) the organic solvent (first organic solvent) included in the raw material solution includes methanol as a main component, (ii) an alcohol having an alkyl group that has 1 to 3 carbon atoms and an alcohol having an alkyl group that has 4 to 5 carbon atoms are combined as the second organic solvent in the basic aqueous solution and the composition thereof is controlled, and (iii) an alcohol having an alkyl group that has 3 to 5 carbon atoms occupies 65 mass% or more of the total mass of all organic solvents (i.e., the first and second organic solvents) excluding water in the basic solution after adding the raw material solution, and thus, the present invention has been completed.

Such a spherical particle forming the silica composite oxide particulate obtained by the production method according to the present invention includes no silica core inside.

Note that the reason why the external color changes depending on the thickness of the HR when the existing silica composite oxide spherical particulate including a silica core is used is not necessarily clear, but the present inventors believe that it is as follows although the present invention is not bound by any logic. That is, it is conceivable that in the case where a silica core is included, the silica core is recognized as a fine particle due to the difference in refractive index between the silica core and the shell that is the surrounding composite oxide, and the transmission light undergoes so-called Rayleigh scattering to scatter the blue light of the incident light (hereinafter, the scattered light will be referred to also as "blue scattered light") when the thickness is thin, which makes it appear bluish. In addition, in a dental curable composition including the silica composite oxide spherical particulate that includes a silica core, the addition amount of red and yellow pigments is often increased to suppress the effects of the blue scattered light. In such a case, the color tone of the pigment develops color more strongly as the thickness of the cured body increases, and the color tone of the cured body appears reddish, which is conceivable to be one of the reasons.

Here, the silica core acts as a nucleus for grain growth and is a particle formed of substantially only silica. The shape and size thereof are not particularly limited, but it has typically a spherical shape and a particle diameter of 0.010 to 0.40 $\mu$m.

Further, it is conceivable that the reason why it has become possible to obtain a particle having a large particle size (particulate having a large average primary particle size) without a silica core by controlling the type of solvent is that the condensable silicon compound and the condensable metal compound react with each other in the raw material solution to form a soluble complex (i.e., an oligomer having both a silicon atom and a metal atom in its structure) and the rate of particle nucleation is slowed down (a large number of nuclei are not allowed to be formed at once) when the raw material solution is added to the basic aqueous organic solution (basic aqueous solution), which enables the grain growth.

As described above, it is very difficult to obtain a particulate that is formed of a spherical particle of a silica composite oxide of a silica-Group 4 type such as a silica-zirconia oxide and has a relatively large particle size, e.g., an average primary particle size of 350 nm or more, such that no silica core is formed. To the best of the present inventors' knowledge, a dental filler formed of a spherical particulate of a silica-Group 4 composite oxide having a large particle size without a silica core is unknown. The dental curable composition according to the present invention has become realizable by the production method according to the present invention that allows a particulate of a silica-Group 4 composite oxide that has a large particle size without a silica core and forms such a dental filler (i.e., the dental filler according to the present invention) to be efficiently produced. In this regard, in the following "2. Details of present invention", (1) Production method according to present invention will be described, and then, (2) Dental filler according to present invention, (3) Dental curable composition according to present invention, and (4) Dental blank for cutting according to present invention will be described in detail.

Note that in the present specification, unless otherwise specified, the evaluation "x to y" using numerical values x and y means x or more and y or less". In such notation, when a unit is assigned only to the numerical value y, the unit is also applied to the numerical value x. Further, in the present specification, the composite oxide means a compound of a plurality of types of oxides, and for each composition thereof, the ratio of the content (moles) of one specific oxide forming the composite oxide with respect to the total amount (moles) of all oxides forming the composite oxide is expressed as the content ratio of the one specific oxide. Further, the term "(meth)acrylic" means both "acrylic" and "methacrylic". Similarly, the term " (meth)acrylate" means both "acrylate" and "methacrylate" and the term "(meth)acryloyl" means both "acryloyl" and "methacryloyl".

2. Details of present invention

(1) Production method according to present invention

(1-1) Overview

The production method according to the present invention is a method of producing a silica composite oxide particulate that includes a composite oxide of silicon and (one or a plurality of types of) metals including at least titanium or zirconium and has a content ratio of silica (silicon oxide) of 80 to 92 (mol%), and includes, as in the case of producing a particulate formed of a coreless spherical particle by the existing so-called sol-gel method, a step of preparing a raw material solution and a step of precipitating a silica composite oxide particulate shown below.

The content ratio of silica as the composition ratio of silicon is expressed as the ratio of the content (moles) of silica with respect to the amount (moles) of all oxides forming the composite oxide.

Step of preparing raw material solution: a step of preparing a raw material solution in which a condensable silicon compound that is a raw material of a silica component; and at least one type of condensable metal compound that is a raw material of a metal oxide component of a silica composite metal oxide forming a spherical particle that is a target product are dissolved in a first solvent including a first organic solvent. Here, the first solvent may include water. Further, in the raw material solution, the condensable silicon compound and the condensable metal compound may react with each other to form a soluble complex. The amount ratio of the silicon atom derived from the condensable silicon compound included in the raw material solution and the at least one type of metal atom derived from the condensable metal compound corresponds to the composition of the silica composite oxide that is a target product. For example, in the raw material solution, a condensable compound such as tetraethyl silicate (condensable silicon compound) and tetra(n-butoxy) zirconate (condensable metal compound) is dissolved.

Step of precipitating silica composite oxide particulate: a step of precipitating, by adding the raw material solution to a basic solution in which a basic compound is dissolved in a second solvent that includes water and a second organic solvent (water-soluble organic solvent) to hydrolyze and condense the condensable silicon compound and the condensable metal compound, a silica composite oxide particulate formed of a spherical particle.

Then, it is characterized in that

(i) as the organic solvent (first organic solvent) excluding water in the raw material solution, an organic solvent having a content ratio of methanol of 80 mass% or more is used,

(ii) as the water-soluble organic solvent (second organic solvent) in the basic solution, a water-soluble organic solvent that can include an alcohol having a short-chain alkyl group that has 1 to 5 carbon atoms, in which the total content ratio of an alcohol having an alkyl group that has 3 carbon atoms and an alcohol having an alkyl group that has 4 to 5 carbon atoms is 80 mass% or more and the content ratio of an alcohol having an alkyl group that has 3 or less carbon atoms is 5 to 50 mass%, is used, and

(iii) an alcohol having an alkyl group that has 3 to 5 carbon atoms occupies 65 mass% or more of the total mass of all organic solvents (first and second organic solvents) excluding water in the basic solution after adding the raw material solution, thereby precipitating, the precipitation step, a silica composite oxide particulate formed of a spherical particle including no silica core (cored formed of only silica) inside, the precipitated particulate having an average primary particle size of 350 to 600 nm.

Note that in the case where a raw material solution including a condensable silicon compound and a condensable metal compound of a general concentration is used, the concentration of organic matters such as alcohols generated by hydrolysis thereof is low, the influence of the organic matter is small, and it is difficult to accurately analyze the concentration of various alcohols in each solution. For this reason, the content ratio of each alcohol specified in the above (i) to (iii) is calculated on the basis of the amount of the organic solvent used as a solvent. Further, the average primary particle size of the silica composite oxide particulate (inorganic particulate or inorganic filler formed of a spherical particle) in the present invention means an average primary particle size determined by image analysis of a scanning electron microscope (SEM) image, and means a value obtained by measuring the maximum diameter (nm) of each of 30 or more particles arbitrarily selected on the basis of an image (or a photograph) obtained by observing the powder sample with a SEM at a magnification of 5,000 to 100,000 times such that 100 or more spherical particles whose entire shapes can be checked are included in the field of view and dividing the sum thereof by the number of particles: n (natural number $\geq$ 30). That is, it means the value defined by

$$x_{AV} = (\Sigma x_i) / n$$

in which $x_i$ (i represents a natural number of 1 to n) represents the maximum diameter of each particle and $x_{AV}$ represents the average primary particle size. Here, when measuring the maximum diameter (nm) of each particle, commercially available image analysis software may be used. It is known that the spherical particles obtained by the so-called sol-gel method have a uniform particle diameter and there are substantially no extremely large or extremely small particles. Since this is also true in the production method according to the present invention, it can be said that the average primary particle size determined by the above method represents the entire average primary particle size.

As described above, in the case where a general sol-gel method is adopted, it is difficult to obtain a silica composite oxide spherical particle having a large particle size without a silica core. On the other hand, in the production method according to the present invention, by controlling the composition of the first organic solvent in the raw material solution, the composition of the second organic solvent (water-soluble organic solvent in the basic solution), and the mixing ratio of the raw material solution and the basic solution, the composition of all organic solvents (first organic solvent and second organic solvent) excluding water in the basic solution after adding the raw material solution is controlled, which allows a spherical particle of a silica composite oxide having a large particle size without a silica core to be efficiently obtained probably because the nucleation rate and grain growth rate are suitably controlled.

As shown in Comparative Example described below, when the above condition (i) is not satisfied and the concentration of methanol in all organic solvents (first organic solvent) excluding water in the raw material solution (hereinafter, referred to also as the "concentration of methanol in the first organic solvent of the raw material solution") is less than 80 mass%, the particles are likely to aggregate and particles with a high degree of uniformity cannot be obtained.

Further, in the case where the water-soluble organic solvent (second organic solvent) in the basic aqueous solution does not satisfy the condition (ii), the particles are likely to aggregate, particles with a high degree of uniformity cannot be obtained, and the particles cannot be caused to grow to 350 nm or more.

Further, in the case where the condition (iii) is not satisfied and the concentration of the alcohol having an alkyl group that has 3 to 5 carbon atoms in all organic solvents (first organic solvent and second organic solvent) excluding water in the basic solution after adding the raw material solution (hereinafter, referred to also as the "concentration of the specific alcohol in the organic solvent of the basic solution after adding the raw material liquid ") is less than 65 mass%, the precipitation of the core particles is promoted, the particle size of the spherical particle of a silica composite oxide becomes small, the particles cannot be caused to grow large, and thus, a particulate having an average primary particle size of 350 nm or more cannot be obtained.

From the viewpoint of effectiveness, the concentration of methanol in the first organic solvent of the raw material solution is favorably 80 to 98 mass%, and the concentration of the specific alcohol in the organic solvent of the basic solution after adding the raw material liquid is favorably 65 to 85 mass%.

In accordance with the production method according to the present invention, for example, it is possible to obtain a particulate formed of the spherical particle with a narrow particle size distribution (having a uniform particle diameter), e.g., the standard deviation value of the average primary particle size of within the range of 1.00 to 1.30, or even the range of 1.00 to 1.25. Further, it is possible to obtain a particulate formed of a spherical particle having a shape close to a perfect sphere, e.g., the average degree of uniformity of 0.6 to 0.7, particularly 0.8 to 0.9.

Note that the standard deviation value of the average primary particle size is defined by the formula: standard deviation value = $\{(x_{AV} + \sigma) / x_{AV}\}$, in which $\sigma$ (nm) represents the standard deviation of the particle diameter of the 30 or more, favorably 100 or more spherical particles in the SEM image used to determine the average primary particle size. The average degree of uniformity is the average value of the ratio of the short diameter to the long diameter of the spherical particle and is defined by the formula: average degree of uniformity = $\{\Sigma(B_i / L_i) / n\}$, in which $L_i$ represents the long diameter that is the maximum diameter measured for the core particles of the n (natural number $\geq$ 30, favorably $\geq$100) spherical particle and $B_i$ represents the short diameter that is the diameter in the direction perpendicular to the long diameter.

Each step of the production method according to the present invention will be described in detail, including the raw material and organic solvent to be used.

(1-2) Details

(A) Step of preparing raw material solution

In the step of preparing a raw material solution, a raw material solution in which a condensable silicon compound that is a raw material of a silica component, which is an oxide of silicon (not a metal); and at least one type of condensable metal compound that is a raw material of a metal oxide component of a silica composite metal oxide forming a spherical particle of a particulate that is a target product are dissolved in a first solvent including a first organic solvent is prepared. The first solvent may include water. In the raw material solution, the condensable silicon compound and the condensable metal compound may react with each other to form a soluble complex. Further, the amount ratio of the silicon atom derived from the condensable silicon compound included in the raw material solution and the at least one type of metal atom derived from the condensable metal compound corresponds to the composition of the particle forming the silica composite oxide particulate that is a target product.

Here, the condensable silicon compound means a compound capable of forming a silica structure (represented by the composition formula: $SiO_2$) by growing a siloxane bond three-dimensionally through polycondensation, and is typically a silicon alkoxide and/or a derivative thereof. For example, in the first composition, a partial hydrolysate of the silicon alkoxide formed under acidic conditions and/or an oligomer obtained by condensing the partial hydrolysate corresponds to the condensable silicon compound. Further, the condensable metal compound means a compound capable of forming a stable structure of an oxide composition in accordance with the type of metal atom by growing a metal atom-oxygen atom bond three-dimensionally through polycondensation, and is typically a metal alkoxide and/or a derivative thereof. For example, in the second composition, at least one selected from the group consisting of a metal alkoxide, a partial hydrolysate thereof, and an oligomer obtained by condensing these corresponds to the condensable metal compound.

In the step of preparing a raw material solution of the production method according to the present invention, in order to efficiently obtain a silica composite oxide particulate having an average primary particle size of 350 to 600 nm without a silica core, it is necessary to set the concentration of methanol in the organic solvent (first organic solvent) of the raw material solution to 80 mass% or more, favorably 85 to 95 mass%.

Note that as another organic solvent (other than methanol) occupying 20 mass% or less, favorably 10 to 18 mass% of all organic solvents (first organic solvent), a polar organic solvent, specifically, an alcohol such as 2-propanol, 1-butanol, and 2-methyl-1-propanol is used. Further, the concentration of the condensable silicon compound in the raw material solution is usually 1.1 to 1.9 mol/l, favorably 1.6 to 1.8 mol/l, expressed in terms of mol/l of the silicon alkoxide.

From the viewpoint of operability, ease of composition control, and the like, the step of preparing a raw material solution favorably includes (a1) a step of preparing, by mixing a silicon alkoxide, methanol, water, and an acid, a first composition (silica component raw material composition) in which a partial hydrolysate of the silicon alkoxide and/or an oligomer (condensable silicon compound) obtained by condensing the partial hydrolysate are dissolved (referred to also as a step of preparing a first composition); (a2) a step of preparing, by mixing a metal alkoxide of (one or a plurality of types of) metals including at least titanium or zirconium and a polar organic solvent for dissolving the metal alkoxide, a second composition (metal oxide component raw material composition) in which at least one (condensable metal compound) selected from the group consisting of the metal alkoxide, a partial hydrolysate thereof, and an oligomer obtained by condensing the partial hydrolysate are dissolved (referred to also as a step of preparing a second composition); and (a3) a step of preparing the raw material solution using the first composition and the second composition (referred to as a step of preparing a raw material solution). Details of each of the above steps will be described below.

(A1) Step of preparing first composition

In the step of preparing a first composition that is a silica component raw material composition, a silicon alkoxide, methanol, water, and an acid are mixed to generate a condensable silicon compound in the solution. The silicon alkoxide used in this step is a compound represented by the formula: $Si(OR)_4$ (in the formula, R represents an alkyl group). In particular, it is favorable to use a silicon alkoxide in which R in the formula represents a methyl group, an ethyl group, an isopropyl group, or a butyl group, because it is easy to control the reactivity and obtain particles having a uniform particle diameter. The silicon alkoxide does not necessarily need to exist as a monomer, and may include a condensate such as a dimer and a trimer.

As the mixing method, it is favorable to add a silicon alkoxide, water, and an acid to methanol held in a container at 35 to 55°C while being stirred. At this time, the amount of methanol is favorably such that the number of moles of the silicon alkoxide per 1 liter of methanol is 2.0 to 3.0 mol/l, particularly 2.5 to 2.9 mol/l. Further, the order of addition and the like are not particularly limited. The silicon alkoxide may be added after adding water and the acid, or water and the acid may be added after adding the silicon alkoxide. Water and the acid may be added separately or may be added in the form of an acidic aqueous solution. As the acid, an inorganic acid such as hydrochloric acid and sulfuric acid is favorable because they are easily available industrially. The molar ratio of water to the silicon alkoxide is favorably within the range of 0.1 to 1 because particles having the above-mentioned average primary particle size can be produced with high productivity. Further, the amount of the acid to be used is favorably such that the amount of protons released by the acid is $2.0 \times 10^{-5}$ to $1.0 \times 10^{-3}$ in molar ratio to the silicon alkoxide. Further, after the addition is completed, it is favorable to continue stirring at 35 to 55°C for approximately 1 to 20 hours. By carrying out the reaction under such conditions, some alkoxide groups of the silicon alkoxide are hydrolyzed to form -OH and these parts are dehydrated (or dealcoholized) and condensed to form a condensable silicon compound formed of a partial hydrolysate and/or an oligomer obtained by condensing the partial hydrolysate.

(A2) Step of preparing second composition

In the step of preparing a second composition that is a metal oxide component raw material, an alkoxide of one or a plurality of types of metals including at least titanium or zirconium and a polar organic solvent for dissolving the metal alkoxide are mixed. In the step, favorable examples of the metal alkoxide including titanium or zirconium include $Ti(OC_3H_7)_4$, $Ti(OC_4H_9)_4$, and $Zr(OC_4H_9)_4$.

In the case where a metal alkoxide other than the metal alkoxide including titanium or zirconium (referred to also as a different metal alkoxide) is combined, examples of the different metal alkoxide that is suitably used include a metal alkoxide of Group 2 or Group 13 of the periodic table, such as $Ba(OC_3H_7)_2$, $Sr(OC_3H_7)_2$, $Ca(OC_3H_7)_2$, and $Al(OC_3H_7)_3$, and an alkali metal alkoxide such as sodium methoxide, sodium ethoxide, lithium methoxide, and lithium ethoxide. The amount of the different metal alkoxide to be combined only needs to be determined appropriately in accordance with the purpose, but is usually 5 to 40 mol%, suitably 10 to 25 mol%, with reference to the total moles of the metal alkoxide including titanium or zirconium and the different metal alkoxide. It is suitable that 8 to 25 mol% of sodium methoxide is included because a spherical particle of a silica composite oxide with few surface acid sites by neutralizing the surface acid sites with sodium ions.

The polar organic solvent for dissolving the metal alkoxide is not particularly limited as long as it is a polar organic solvent that dissolves the metal alkoxide to be actually used, but an alcohol other than methanol, such as 2-propanol, 1-butanol, and 2-methyl-1-propanol, is suitably used because they have high solubility for the metal alkoxide including titanium or zirconium. Note that in the case where a plurality of types of metal alkoxides is used, the plurality of types of metal alkoxides may be dissolved in a common polar organic solvent, or each of them may be dissolved in a polar organic solvent (as necessary, a different polar organic solvent) in advance and then mixed. Further, the polar organic solvent that is a solvent for the metal oxide component raw material composition may include water, favorably does not include an acid, and favorably does not include water (excluding water that is inevitably contained, such as an impurity) or an acid.

The concentration of the metal alkoxide included in the metal oxide component raw material composition is usually 1.1 to 1.9 mol/l, favorably 1.2 to 1.8 mol/l, expressed in terms of total mol/l of the metal alkoxide.

Note that in the case where the different metal alkoxide is used, the different metal alkoxide does not necessarily need to be a metal oxide component raw material composition, and may be added directly to the mixture of the silica component raw material composition and the metal oxide component raw material composition obtained in the step of preparing a raw material solution.

(A3) Step of preparing raw material solution

In the step of preparing a raw material solution, a raw material solution is prepared by using the first composition (silica component raw material composition) and the second composition (metal oxide component raw material composition), specifically, mixing both compositions. The method of mixing both the compositions is not particularly limited, but it is favorable to adopt a method of adding the second composition to the first composition while being stirred and mixing them. The amounts of both the compositions to be mixed only need to be determined in accordance with the composition of (spherical particle forming) the target silica composite oxide particulate, and are adjusted in accordance with the concentration of the first and the second composition (e.g., the concentration of the silicon alkoxide and the metal alkoxide to be blended).

When mixing, the concentration of methanol in the organic solvent (first organic solvent) of the raw material solution in the obtained raw material solution needs to be 80 mass% or more, favorably 85 to 95 mass%. When the concentration of methanol is 80 mass% or more, it is considerable that the condensable silicon compound (condensable silica component raw material compound) and the condensable metal compound (condensable metal oxide component raw material compound) react with each other in the raw material solution to form a soluble complex, i.e., an oligomer having both a silicon atom and a metal atom in its structure, thereby suppressing the nucleation during the precipitation step and the separation of the condensable silicon compound and the condensable metal compound during the grain growth process.

Since the composition of the target silica composite metal oxide has a silica content ratio of 80 to 92 (mol%), the concentration of methanol in the first organic solvent of the raw material solution is approximately 80 mass% or more when the concentration of alkoxide in both of the compositions are similar. However, when the concentration of methanol after mixing both of the compositions on the basis of the composition ratio is less than 80 mass%, it only needs to be adjusted by newly adding methanol. As the addition of methanol, the necessary amount of methanol, which is obtained by calculation, may be added to the first composition in advance or may be added after mixing.

After adding the condensable metal compound (and the different metal alkoxide added directly as necessary), it is favorable to stir for approximately 5 to 30 minutes.

(B) Precipitation step

In the precipitation step, the raw material solution is added to a basic solution including a second organic solvent that is compatible with water (water-soluble organic solvent), water, and a basic compound, and a spherical particle is precipitated by nucleation and grain growth, thereby obtaining a silica composite oxide particulate that is formed of a silica composite oxide having the target composition and has an average primary particle size of 350 to 600 nm. At this time, in order to efficiently obtain a silica composite oxide particulate having an average primary particle size of 350 to 600 nm, a water-soluble organic solvent (second organic solvent) that includes an alcohol having a short-chain alkyl group that has 1 to 5 carbon atoms, in which the total content ratio of an alcohol having an alkyl group that has 3 carbon atoms and an alcohol having an alkyl group that has 4 to 5 carbon atoms is 80 mass% or more, favorably 85 mass% or more, and the content of an alcohol having an alkyl group that has 3 or less carbon atom is 5 to 50 mass%, favorably 6 to 40 mass%, is used as a water-soluble organic solvent (second organic solvent) in the basic solution, and an alcohol having an alkyl group that has 3 to 5 carbon atoms (hereinafter, also referred to simply as "specific alcohol") is caused to occupy 65 mass% or more of the total mass of all organic solvents (first and second organic solvents) excluding water in the basic solution after adding the raw material solution.

In order to satisfy the above conditions, the water-soluble organic solvent (second organic solvent) to be used when preparing the basic solution needs to combine an alcohol having an alkyl group that has 3 or less carbon atom and an alcohol having an alkyl group that has 4 to 5 carbon atoms. The water-soluble organic solvent may include a water-soluble organic solvent other than an alcohol having an alkyl group that has 1 to 5 carbon atoms, but typically includes an alcohol having a short-chain alkyl group that has 1 to 5 carbon atoms, favorably includes a specific alcohol as well as methanol and/or ethanol, and more favorably includes only a specific alcohol (that is, it is more favorable that the second organic solvent is the specific alcohol). As the specific alcohol, 1-propanol, 2-propanol, 1-butanol, 2-methyl-1-propanol, 1-pentanol, or the like is suitably used.

As a basic compound used for the basic solution, ammonia and sodium hydroxide are suitably used and ammonia (water) is particularly suitably used. The concentration of the basic component (basic compound) in the basic mixed solution is favorably within the range of 15 to 20 mol%, and the concentration of water is favorably 15 to 25 mass%.

In the precipitation step, usually, no solution other than the raw material solution and no solvent are added, and only the raw material solution is added to the basic solution. For this reason, the amount of the basic solution to be used only needs to be the amount that makes the concentration of the specific alcohol in the organic solvent of the basic solution after adding the raw material liquid is 65 mass% or more in accordance with the amount of the specific alcohol in the basic solution, the amount of the raw material solution to be added, and the amount and composition of the organic solvent in the raw material solution, and an excess amount of the basic solution may be used. However, it is favorable to obtain the amount that makes a predetermined concentration by calculation and use the amount.

The method of adding the raw material solution to the basic solution in the precipitation step is not particularly limited. However, the method of adding the hydrolysable raw material solution in small amounts in a continuous manner to the basic mixed solution being stirred with a stirring blade, a stirrer, or the like is favorable because it is easy to obtain particles with a uniform particle diameter.

In the precipitation step, the average primary particle size can be controlled by the temperature of the basic solution and the time for adding the raw material solution dropwise. That is, from the viewpoint of improving the degree of uniformity of the particle diameter, the temperature of the basic solution is favorably 35 to 55°C, and the particle diameter can be made smaller as the temperature is higher. The longer the time for adding the raw material solution dropwise, the better the particle shape tends to be. Further, the longer the dropping time, the larger the particle diameter can be made. Considering the above, it is favorable to set the temperature of the reaction tank to 40 to 50°C and the dropping time to 1 to 9 hours because it is possible to obtain a particulate having an average primary particle size of 350 to 600 nm with high productivity.

After the precipitation step is completed, it only needs to collect the particulate precipitated by decantation or filtration and dry the collected particulate.

**(C) Other steps**

In the production method according to the present invention, in order to remove the solvent from the particulate and reduce the hydroxy groups on the particle surface, a firing step of firing the particulate may be performed. At this time, the firing temperature is favorably 700 to 1500°C because a particulate having a denser crystal structure can be obtained.

Further, in order to improve the affinity between the silica composite oxide particulate and the organic matrix of the dental curable composition, the particle may be subjected to silane coupling treatment. As the silane coupling agent used in the silane coupling treatment, any known silane coupling agent can be used without any restrictions. Further, in order to reduce the effect of strong acid sites on the particle surface, the surface may be covered with silica.

**(2) Dental filler according to present invention**

The silica composite oxide particulate that is formed of a spherical particle of a silica composite oxide obtained by the production method according to the present invention and has an average primary particle size of 350 to 600 nm is suitably used as a dental filler (filler).

In the dental filler according to the present invention, since the silica composite oxide particulate has an average primary particle size (corresponding to the average particle diameter of the particulate) of 350 to 600 nm, the filling rate of the inorganic filler when used in combination with inorganic particles having an average particle diameter of less than 100 nm increases and the strength of the cured body is improved. In the case where the average particle diameter is less than 350 nm, there is a possibility that stringiness or stickiness easily occurs when mixed with a polymerizable monomer to form a composition, and it is difficult to blend into the composition at a high filling rate. Further, in the case where the average particle diameter exceeds 600 nm, there is a possibility that the polishing properties decrease. From the viewpoint of achieving higher strength, the average particle diameter is favorably 350 to 550 nm.

Each of the spherical particles forming the dental filler according to the present invention only needs to have a substantially spherical shape and does not necessarily need to have a perfect spherical shape. In general, it only needs to have the average degree of uniformity of 0.6 or more, favorably 0.8 or more.

The silica composite oxide forming the dental filler according to the present invention allows radiopacity that is favorable as a dental curable composition to be provided and the refractive index to be adjusted to a favorable one. Note that by blending the metal oxide within the above range, the refractive index of the obtained silica composite oxide particulate can be adjusted to a range of 1.50 to 1.58.

Further, the dental filler according to the present invention is favorably used as a filler for a dental curable composition that includes a radically polymerizable monomer component formed of the following (meth)acrylic compound. That is, it is favorably used as a filler for a dental curable composition in which the radically polymerizable monomer component is formed of (only) the (meth)acrylic compound that provides a cured body satisfying the formula: $0.01 < n_F - n_P < 0.1$, in which $n_F$ represents a refractive index of the spherical particle of a silica composite oxide forming the silica composite oxide particulate at 25°C and with respect to light of a wavelength of 589 nm and $n_P$ represents a refractive index of the cured body of the (meth)acrylic compound at 25°C and with respect to light of a wavelength of 589 nm.

In the case where the dental filler according to the present invention is used for such a dental curable composition, a color shift is unlikely to occur even if the thickness of the cured body changes. For example, a cured sample A having a thickness of 1 mm, which is formed of a cured body of a homogenous curable composition including the (meth)acrylic compound: 100 parts by mass, the dental filler according to the present invention: 200 parts by mass, and a photopolymerization initiator: the amount necessary for curing, and a cured sample B having a thickness of 3 mm are prepared, and when each of them is measured using a color difference meter, the relationship of the following formula is satisfied:

$$\text{Formula: } 0.8 \leq (R_B \, / \, R_A) < 2.0$$

in which $R_A$ represents the spectral reflectance of the sample A and $R_B$ represents the spectral reflectance of the sample B at a wavelength of 450 nm.

**(3) Dental curable composition according to present invention**

**(3-1) Overview**

The dental curable composition according to the present invention is a dental curable composition that includes a radically polymerizable monomer: 100 parts by mass and a silica composite oxide particulate: 100 to 800 parts by mass formed of a spherical particle of a silica composite oxide that is formed of a silica composite oxide that is a composite oxide of silicon and (one or a plurality of types of) metals including at least titanium or zirconium and has an average primary particle size of 350 to 600 nm, and is characterized in that the silica content ratio, which is defined as the ratio of the content (moles) of silica in the silica composite oxide to the amount (moles) of all oxides forming the silica composite oxide, is 80 to 92 (mol%) and the silica composite oxide spherical particle includes no core (silica core) formed of only silica inside the particle. Then, by satisfying such conditions, it is possible to prevent a color shift (changes in the blue color and red color of the external color tone) of the cured body due to the difference in thicknesses when cured from occurring.

The particulate formed of the spherical particle of a silica composite oxide is produced by the production method according to the present invention, the silica composite oxide particulate corresponds to the dental filler according to the present invention, and the details thereof is as described above. In this regard, now, other components forming the dental curable composition according to the present invention will be mainly described.

(3-2) Details

(A) Radically polymerizable monomer

As the radically polymerizable monomer, a (meth)acrylic compound radically polymerizable monomer such as a (meth)acrylic compound that can be used in a dental curable composition; a cationic polymerizable monomer such as epoxies and oxetanes; and the like can be used without particular restrictions, but the (meth)acrylic compound is favorably used. Examples of the polymerizable monomer that is suitably used include methyl (meth)acrylate, ethyl (meth)acrylate, n-butyl (meth)acrylate, 2-ethylhexyl (meth)acrylate, (meth)acrylic acid, N-(meth)acryloylglycine, N-(meth)acryloyl aspartic acid, N-(meth)acryloyl-5-aminosalicylic acid, 2-hydroxyethyl (meth)acrylate, 3-hydroxypropyl (meth)acrylate, 4-hydroxybutyl (meth)acrylate, 6-hydroxyhexyl (meth)acrylate, 2,2-bis[(3-methacryloyloxy-2-hydroxypropyloxy)phenyl]propane, 2,2-bis(4-methacryloyloxyphenyl)propane, 2,2-bis(4-methacryloyloxypolyethoxyphenyl)propane, triethylene glycol dimethacrylate, tetraethylene glycol dimethacrylate, neopentyl glycol dimethacrylate, trimethylolpropane trimethacrylate, and pentaerythritol tetramethacrylate.

As the radically polymerizable monomer component, generally, a plurality of types of radically polymerizable monomers is used. In this case, setting the type and mixing amount of the polymerizable monomer such that the absolute value of the difference between the refractive index of the organic matrix of the cured body obtained by curing the dental curable composition according to the present invention and the refractive index of the spherical particle of a silica composite oxide is within 0.1 is favorable from the viewpoint of reducing light scattering due to the difference in refractive index between the organic matrix-silica composite oxide spherical particle to thereby make the transparency of the cured body closer to that of natural tooth substance. Specifically, it is favorable to use a radically polymerizable monomer formed of a (meth)acrylic compound that provides a cured body satisfying the formula: $0.01 < n_F - n_P < 0.1$ in which $n_F$ represents a refractive index of the spherical particle of a silica composite oxide forming the silica composite oxide particulate at 25°C and with respect to light of a wavelength of 589 nm and $n_P$ represents a refractive index of a cured body of the radically polymerizable monomer formed of a (meth)acrylic compound at 25°C and with respect to light of a wavelength of 589 nm.

(B) Silica composite oxide particulate formed of spherical particle

The silica composite oxide particulate is used for the dental filler according to the present invention. The content of the silica composite oxide particulate only needs to be within the range of 100 to 800 parts by mass with respect to 100 parts by mass of the radically polymerizable monomer component, but is favorably 100 to 600 parts by mass, more favorably 200 to 500 parts by mass, from the viewpoint of making the fluidity of the curable composition in a suitable range.

(C) Polymerization initiator

As the polymerization initiator, any known polymerization initiator can be used without particular restrictions. Among them, it is favorable to use a photopolymerization initiator and a thermal polymerization initiator.

As the photopolymerization initiator, a combination of a photosensitizing compound and a tertiary amine is favorable. Of these, an α-diketone such as camphorquinone, 9,10-phenanthrenequinone, benzyl, diacetyl, acetylbenzoyl, 2,3-pentadione, 2,3-octadione, and 4,4'-dimethoxybenzyl, acenaphthenequinone is favorable as the photosensitizing compound, and N,N-diethyl-p-toluidine, p-(N,N-dimethyl)aminomethyl benzoate, p-(N,N-dimethyl)aminoethyl benzoate, triethanolamine, or N-methyl diethanolamine is favorable as the tertiary amine compound.

As the thermal polymerization initiator, a peroxide such as benzoyl peroxide, p-chlorobenzoyl peroxide, tert-butylperoxy-2-ethylhexanoate, tert-butyl peroxydicarbonate, diisopropyl peroxydicarbonate is favorable. A thermal polymerization initiator such as an azo compound such as azobisisobutyronitrile, a boron compound such as tributylborane, a tributylborane partial oxide, sodium tetraphenylborate, sodium tetrakis(p-fluorophenyl)borate, and a tetraphenylborate triethanolamine salt, barbituric acids such as 5-butylbarbituric acid and 1-benzyl-5-phenylbarbituric acid, and sulfinates such as sodium benzenesulfinate, and sodium p-toluenesulfinate is suitably used. These polymerization initiators may be used alone or two or more of them may be mixed and used.

(D) Other additives

The curable composition according to the present invention is favorably toned by blending a dye such as a pigment commonly used in dental applications such that the cured body will have a predetermined color tone, and then used. In the case where a silica composite oxide particulate including a silica core is used, it has been necessary to blend a pigment component to counteract the effect of blue scattered light derived from the particle in accordance with the thickness of the cured body during use. However, in the curable composition according to the present invention, it is unnecessary to blend a pigment component for such a purpose and the desired color tone (after toning) is unlikely to change depending on the thickness of the cured body. The blending amount of the dye such as a pigment is usually 800 to 8000 ppm by mass with reference to the total mass of the dental curable composition. In the case where a cured body of the curable composition according to the present invention is used as a to-be-cut portion of a dental blank for cutting, it is favorable to provide cured bodies toned such that the color tones of the cured bodies are some shades selected from the above 16 shades.

Further, another additive such as a polymerization inhibitor and an ultraviolet absorber may be included within a range that does not impair the effect of the present invention.

(4) Dental blank for cutting according to present invention

The dental blank for cutting according to the present invention is characterized in that the change in the external color between the blank form and the form after cutting is suppressed by using the cured body of a dental curable composition as a to-be-cut portion (i.e., by including a to-be-cut portion that includes the cured body of the dental curable composition according to the present invention).

The blank according to the present invention is not particularly different from the existing HR blank except for the above feature point, and may include a holding member such as a holding pin for fixing to a cutting machine, as necessary. Further, the shape and size of the to-be-cut portion are not particularly limited, and may be a (solid) block molded into a rectangular parallelepiped shape or a columnar shape or a (solid) disk formed into a plate shape or disk shape.

Further, at least part of the to-be-cut portion only needs to be formed of a cured body of the dental curable composition according to the present invention. For example, the to-be-cut portion may have a so-called stacked structure in which another HR blank is staked on the cured body of the dental curable composition according to the present invention.

(Examples)

Although the present invention will be further specifically described below by way of Examples, the present invention is not limited to these Examples.

1. Examples, Comparative Examples, and Reference Examples regarding production of silica composite oxide particulate

The abbreviations of the compounds used in the production of the silica composite oxide particulate are shown below.

<Condensable silicon compound> (referred to also as a Si oxide raw material)

· TES: tetraethyl silicate

<Condensable metal compound>(referred to also as a metal oxide raw material)

   · TBZ: tetra(n-butoxy)zirconate
   · TiPT: tetra(isopropoxy)titanate
   · NaOMe: sodium methoxide

<First and second (water-soluble) organic solvents>

· MeOH: methanol

Alcohol having alkyl group that has 3 carbon atoms (referred to also as a C3 alcohol)

· IPA: 2-propanol

Alcohol having alkyl group that has 4 carbon atoms (referred to also as a C4 alcohol)

· NBA: 1-butanol

· IBA: 2-methyl-1-propanol

· TBA: 2-methyl-2-propanol

Example 1 [Production and evaluation of silica composite oxide particulate F1]

(I) Production of silica composite oxide particulate F1

10 g of 0.05 mass%-hydrochloric acid and 300 g of TES (manufactured by Colcoat Co,.Ltd.) that is a Si oxide raw material were dissolved in 400 g of MeOH and mixed at 40°C for 4 hours to hydrolyze, thereby preparing a first composition (abbreviated as "Si raw material solution " in Table 1). Subsequently, 80 g of TBZ (manufactured by NIPPON SODA CO., LTD.) that is a metal oxide raw material and 50 g of IBA that is a C4 alcohol serving as a water-soluble organic solvent were mixed to prepare a solution 1 that is a second composition (abbreviated as "metal raw material solution" in Table 1). Then, the second composition was added to the first composition and stirred for 10 minutes to obtain a mixed solution, and 9 g of a methanol solution (solution 2) with the concentration of NaOMe of 28 mass% was added as a second composition while being stirred to prepare a raw material solution.

Next, 320 g of a 25 mass% ammonia aqueous solution was added to 100 g of IPA that is a C3 alcohol and 700 g of IBA that is a C4 alcohol, which were placed in a 3L glass reaction vessel with a stirrer, to prepare a basic solution including an ammoniacal alcohol solution.

After that, the raw material solution prepared previously was added to the basic solution over a period of 6 hours while maintaining the temperature of the reaction vessel at 45°C, and the mixture was stirred for 30 minutes after the addition was completed to obtain a silica composite oxide particulate.

Subsequently, the particulate was collected by suction filtration and dried under a reduced pressure at 80°C to obtain a white powder. The powder was fired at 800°C for 4 hours and the obtained powder was used as a silica composite oxide particulate F1 (hereinafter, referred to as the particle F1).

Th conditions in the above method are summarized in Table 1. The numerical value in the column of the raw material in Table 1 indicates the amount (unit: g) of each raw material used. As shown in Table 1, the content ratio of methanol in the organic solvent (first organic solvent) in the raw material solution is 89.0 mass%, the total content ratio of an alcohol having an alkyl group that has 3 carbon atoms and an alcohol having an alkyl group that has 4 to 5 carbon atoms included in the water-soluble organic solvent (second organic solvent) in the basic solution is 100 mass%, and the content ratio of an alcohol having an alkyl group that has 3 or less carbon atom is 12.5 mass%, and the content ratio of an alcohol having an alkyl group that has 3 to 5 carbon atoms in all organic solvents (first and second organic solvents) excluding water in the basic solution after adding the raw material solution is 67.6 mass%. Note that "↑" in Table means "same as above".

(II) Evaluation of silica composite oxide particulate F1

The average primary particle size, the standard deviation value of the particle diameter, the average degree of uniformity, and the refractive index of the silica composite oxide particulate F1 (particle F1) obtained by the above method were evaluated by the methods described below. The results are shown in Table 2.

<Method of measuring average primary particle size of silica composite oxide particulate, standard deviation value of particle diameter, and average degree of uniformity>

The average primary particle size: $x_{AV}$ (nm) of the silica composite oxide particulate was calculated by performing image processing on the photograph of powder taken by a scanning electron microscope ("XL-30S", manufactured by PHILIPS) at a magnification of 5,000 to 100,000 times using image analysis software ("IP-1000PC", manufactured by Asahi Kase Engineering Corporation), measuring the maximum diameter: $x_i$ (unit: nm. i means a natural number of 1 to n) of each of n (wherein, n means a natural number of 30 or more) randomly selected from 100 or more particles observed within a unit field of view, and using, from the sum thereof: $\Sigma x_i$, the following formula:

$$x_{AV} = (\Sigma x_i) / n.$$

Further, the standard deviation value of the average primary particle size was calculated as follows. For the 30 or more spherical particles in the SEM image used to determine the average primary particle size, a standard deviation $\sigma$ (nm) of the particle diameter was obtained from the sum $(\Sigma(x_{AV} - x_i)^2)$ of the squares of the difference between the maximum diameter $x_i$ of each particle and the average primary particle size $x_{AV}$ using the following formula:

$$\sigma = [\{\Sigma(x_{AV} - x_i)^2\} / n]^{1/2},$$

and
the standard deviation value was obtained using the following formula:

$$\text{Standard deviation value} = \{(x_{AV} + \sigma) / x_{AV}\}.$$

Further, the average degree of uniformity was calculated as follows. For each of the n (natural number of $\geq 30$) spherical particles, the average degree of uniformity was obtained from the sum $(\Sigma(B_i / L_i))$ of the ratios $(B_i / L_i)$ of the following two in each particle using the following formula:

$$\text{Average degree of uniformity} = \{\Sigma(B_i / L_i) / n\}$$

in which $L_i$ represents the long diameter that is the measured maximum diameter and $B_i$ represents the short diameter that is the diameter in the direction perpendicular to the long diameter.

<Method of measuring refractive index: nF of silica composite oxide spherical particle>

The refractive index of the particle forming the silica composite oxide particulate was measured by a liquid immersion method (measurement wavelength: 589 nm) using an Abbe refractometer (manufactured by ATAGO CO.,LTD.) That is, in a thermostatic chamber at 25°C, 1 g of the silica composite oxide particulate was dispersed in 50 mL of anhydrous toluene in a 100 mL sample bottle. 1-bromotoluene was added dropwise little by little while stirring this dispersion liquid with a stirrer, the refractive index: nF of the dispersion liquid at the time point when the dispersion liquid became most transparent was measured, and the obtained value was used as the refractive index of the spherical particle of a silica composite oxide.

Examples 2 to 5 and 7 and Comparative Examples 1 to 4 [Production and evaluation of silica composite oxide particulates F2 to F5 and F7 to F11]

Silica composite oxide particulates F2 to F5 and F7 to F11 (hereinafter, respectively referred to as particles F7 to F11 in some cases) were obtained in the same manner as that in Example 1 except that the raw material and the amount of the raw material used were changed as shown in Table 1. The obtained silica composite oxide particulates were evaluated in the same manner as that in Example 1. The results are shown in Table 2.

Example 6 [Production and evaluation of silica composite oxide particulate F6]

A silica composite oxide particulate was synthesized in the same manner as that in Example except that the raw material and the amount of the raw material used were changed as shown in Table 1. Subsequently, the particulate was collected by suction filtration and dried under a reduced pressure at 80°C to obtain a white powder. The powder was fired at 700°C for 4 hours and the obtained powder was used as a silica composite oxide particulate F6. The obtained silica composite oxide particulate F6 (hereinafter, referred to also as a particle F6 in some cases) was evaluated in the same manner as that in Example 1. The results are shown in Table 2.

Reference Example 1 [Production and evaluation of silica composite oxide particulate F12 having silica core structure]

10 g of 0.05 mass% hydrochloric acid and 270 g of TES (manufactured by Colcoat Co,.Ltd.) were dissolved in 400 g of MeOH and mixed at 40°C for 4 hours to hydrolyze to obtain a first composition (silica component raw material composition). Subsequently, 80 g of TBZ (manufactured by NIPPON SODA CO., LTD.) and 50 g of IBA were mixed to obtain a second composition (metal oxide component raw material composition). Then, the second composition was added to the first composition and stirred for 10 minutes. Subsequently, 9 g of the methanol solution with the concentration of NaOMe 28 mass% was added to the obtained mixed solution as a second composition while being stirred to obtain a raw material solution.

Next, a 3L glass reaction vessel with a stirrer was filled with 700 g of IBA and 100 g of IPA and 320 g of a 25 mass% ammonia aqueous solution was added thereto, thereby preparing an ammoniacal alcohol solution. 30 g of TES was added to this solution while maintaining the temperature of the reaction vessel at 45°C and stirred for 30 minutes to form a silica core particle in the reaction vessel. The raw material solution prepared previously was added thereto over a period of 6 hours while maintaining the temperature of the reaction vessel at 45°C, and the mixture was stirred for 30 minutes after the addition was completed to obtain a silica composite oxide particulate. Subsequently, the particulate was collected by suction filtration and dried under a reduced pressure at 80°C to obtain a white powder. The powder was fired at 800°C for 4 hours and the obtained powder was used as a silica composite oxide particulate F12 (hereinafter, referred to also as a particle F12). The obtained silica composite oxide particulate F12 having a silica core was valuated in the same manner as that in Example 1. The results are shown in Table 2.

(Table 1)

| No. | | Particle abbreviation | Step of preparing Si raw material solution: Composition of Si raw material solution (① Si oxide raw material (g), ② Organic solvent (g), ③ Acid aqueous solution (g)) | Step of preparing raw material solution: Composition of metal raw material solution (① Metal oxide raw material (g), ② Organic solvent (g)) Solution 1 | Solution 2 | Concentration of MeOH in organic solvent (mass%) | Step of preparing basic solution: Composition of water-soluble organic solvent C1-3 alcohol (g) | C4-5 alcohol (g) | C1-3 alcohol content ratio (mass%) | C3-5 alcohol content ratio (mass%) | Precipitation step: Specific alcohol content ratio after adding raw material liquid (mass%) |
|---|---|---|---|---|---|---|---|---|---|---|---|
| Example | 1 | F1 | ① TES (300), ② MeOH (400), ③ HCl aqueous solution (10) | ① TBZ (80), ② IBA (50) | ① NaOMe (2.5), ② MeOH (6.5) | 89.0 | IPA (100) | IBA (700) | 12.5 | 100.0 | 67.6 |
| | 2 | F2 | ← | ← | ← | ← | IPA (200) | NBA (600) | 25.0 | ← | ← |
| | 3 | F3 | ← | ← | | ← | ← | TBA (600) | ← | ← | ← |
| | 4 | F4 | ① TES (240), ② MeOH (400), ③ HCl aqueous solution (8) | ① TBZ (83), ② IBA (50) | | ← | IPA (100) | IBA (700) | 12.5 | ← | ← |
| | 5 | F5 | ← | ① TBZ (95), ② IBA (50) | | ← | ← | ← | ← | ← | ← |
| | 6 | F6 | ① TES (300), ② MeOH (400), ③ HCl aqueous solution (10) | ① TiPr (55), ② IBA (50) | | ← | ← | ← | ← | ← | ← |
| | 7 | F7 | ← | ① TBZ (80), ② IBA (50) | | ← | MeOH (70) | IBA (880) | 7.4 | 92.6 | 66.1 |
| Comparative Example | 1 | F8 | ① TES (300), ② MeOH (200), IPA (200), ③ HCl aqueous solution (10) | ← | ← | 45.3 | IPA (100) | IBA (700) | 12.5 | 100.0 | 83.6 |
| | 2 | F9 | ① TES (300), ② MeOH (400), ③ HCl aqueous solution (10) | ← | ← | 89.0 | — | IBA (800) | 0.0 | ← | 67.6 |
| | 3 | F10 | ← | ← | ← | ← | IPA (700) | IBA (100) | 87.5 | ← | ← |
| | 4 | F11 | ← | ← | ← | ← | MeOH (100) | IBA (700) | 12.5 | 87.5 | 59.7 |
| Reference Example 1 | | F12* | ① TES (270), ② MeOH (400), ③ HCl aqueous solution (10) | ← | ← | ← | IPA (100) | IBA (700) | ← | 100.0 | 67.6 |

*In Reference Example 1, 30 g of TES is added before adding the raw material solution to the basic solution to form a silica core. Therefore, F12 has a silica core. The other F1 to F11 have no silica core.

(Table 2)

| No. | Particle abbreviation | Average primary particle size [nm] | Standard deviation value | Average degree of uniformity | Composition (mol%) $SiO_2/ZrO_2/TiO_2/Na_2O$ | Refractive index: $n_F$ |
|---|---|---|---|---|---|---|
| Example 1 | F1 | 542 | 1.15 | 0.90 | 87.6/11.0/0/1.4 | 1.526 |
| Example 2 | F2 | 404 | 1.21 | 0.86 | 87.6/11.0/0/1.4 | 1.526 |
| Example 3 | F3 | 457 | 1.24 | 0.81 | 87.6/11.0/0/1.4 | 1.526 |
| Example 4 | F4 | 425 | 1.28 | 0.83 | 84.6/13.7/0/1.7 | 1.539 |
| Example 5 | F5 | 441 | 1.26 | 0.82 | 82.8/15.5/0/1.7 | 1.548 |
| Example 6 | F6 | 383 | 1.16 | 0.85 | 87.4/0/11.2/1.4 | 1.538 |
| Example 7 | F7 | 360 | 1.21 | 0.84 | 87.6/11.0/0/1.4 | 1.526 |
| Comparative Example 1 | F8 | 450 | 1.44 | 0.58 | 87.6/11.0/0/1.4 | 1.526 |
| Comparative Example 2 | F9 | 466 | 1.45 | 0.59 | 87.6/11.0/0/1.4 | 1.526 |
| Comparative Example 3 | F10 | 289 | 1.20 | 0.85 | 87.6/11.0/0/1.4 | 1.526 |
| Comparative Example 4 | F11 | 250 | 1.08 | 0.92 | 87.6/11.0/0/1.4 | 1.526 |
| Reference Example 1 | F12 | 422 | 1.10 | 0.90 | 87.6/11.0/0/1.4 | 1.527 |

It can be said that in the particles F1 to F7 produced by the production method according to the present invention, there is little adhesion and aggregation between particles because the standard deviation value is within 1.30 and the average degree of uniformity satisfies the substantially spherical shape. In contrast, the silica composite oxide particulates F8 and F9 produced under conditions that did not satisfy the conditions of the production method according to the present invention showed a large variation in a standard deviation value and a large average degree of uniformity. From this, it can be seen that adhesion between particles has occurred. Further, the silica composite oxide particulates F10 and F11 produced under conditions that did not satisfy the conditions of the production method according to the present invention had a substantially spherical shape, but the average primary particle size thereof did not reach 350 nm.

2. Examples and Comparative Examples regarding (non-colored) dental curable composition

The abbreviations of the compounds used in the preparation of the dental curable composition are shown below.

· UDMA: 1,6-bis(methacrylethyloxycarbonylamino)trimethylhexane
· Bis-GMA: 2,2-bis[(3-methacryloyloxy-2-hydroxypropyloxy)phenyl]propane
· 3G: triethylene glycol dimethacrylate
· CQ: camphorquinone
· DMBE: p-(N,N-dimethyl)aminoethyl benzoate
· HQME: hydroquinone monomethyl ether.

Note that when preparing the dental curable composition, monomer compositions (referred to also as matrixes) M1 to M3 having the compositions shown in Table 3 were prepared in advance and used. Further, the silica composite oxide particulates F1 to F12 used for preparing the dental curable composition were subjected to surface treatment with γ-methacryloyloxypropyltrimethoxysilane to make the surface hydrophobic, and then added to the matrixes and mixed. Note that the refractive index before and after curing in Table 3 was measured as follows.

<Measurement of refractive index before curing>

Measurement was performed in a thermostatic chamber at 25°C using an Abbe refractometer (manufactured by ATAGO CO.,LTD.) (measurement wavelength: 589 nm).

<Measurement of refractive index: nP after curing>

0.2 mass% camphorquinone, 0.3 mass% p-(N,N-dimethyl)aminoethyl benzoate, and 0.15 mass% hydroquinone monomethyl ether were mixed with each monomer composition and homogenized, the homogenized mixture was placed in a mold with a hole of 7 mm$\varphi \times$ 0.5 mm, and both surfaces were pressed with a polyester film. After that, light was applied thereto for 30 seconds to cure it using a halogen-type dental light irradiator ("Demetron LC" manufactured by Cybron) with a light intensity of 500 mW/cm$^2$, and then, it was removed from the mold, thereby preparing a cured sample. When a sample was setting in an Abbe refractometer (manufactured by ATAGO CO.,LTD.), a solvent (bromonaphthalene) that does not dissolve the sample and has a refractive index higher than that of the sample was added dropwise to the sample to bring the sample into close contact with the measurement surface, and the refractive index: nP was measured in the same manner as that for the refractive index before curing.

(Table 3)

| Matrix | Composition of polymerizable monomer | Refractive index | |
|---|---|---|---|
| | | Before curing | After curing: $n_P$ |
| M1 | UDMA (80) /3G (20) | 1.493 | 1.510 |
| M2 | Bis-GMA (30) / UDMA (30) / 3G (40) | 1.507 | 1.528 |
| M3 | Bis-GMA (40) / UDMA (30) / 3G (30) | 1.515 | 1.533 |

Example 8 [Preparation and evaluation of dental curable composition CR1]

Under red light, 0.6 parts by mass of CQ, 1.0 part by mass of DMBE, and 0.15 parts by mass of HQME were added to 100 parts by mass of a matrix M1 and mixed to prepare a homogenous polymerizable monomer composition. 200 parts by mass of the silica composite oxide particulate F1 (that has been subjected to surface treatment) was weighed out and placed in a mortar, and the polymerizable monomer composition was gradually added thereto under red light and sufficiently kneaded with a pestle to obtain a homogenous curable paste. Further, this paste was degassed under reduced pressure to remove air bubbles to produce a dental curable composition CR1. The spectral reflectance of the obtained CR1 was measured on the basis of the evaluation method shown below. The composition and evaluation results are shown in Table 4.

<Method of measuring spectral reflectance of cured body of dental curable composition>

The prepared dental curable composition CR1 was placed in molds with holes of 7 mm$\varphi \times$ 1 mm thickness and 3 mm thickness, and both surfaces thereof were pressed with a polyester film. After that, light was applied thereto for 30 seconds to cure it using a halogen-type dental light irradiator ("Demetron LC" manufactured by Cybron) with a light intensity of 500 mW/cm$^2$, and then, they were removed from the molds. The surfaces thereof were polished to a mirror finish to obtain a cured sample A having a thickness of 1 mm and a cured sample B having a thickness of 3 mm. The spectral reflectance of each of the samples using a spectrophotometer ("TC-1800MKII" manufactured by TokyoDenshoku.co.,Ltd., a halogen lamp: 12V100W, a measurement wavelength range of 380 to 780 nm) against a black background using a black carbon tape to obtain a spectral reflectance curve. The spectral reflectance R of the spectral reflectance curve at a wavelength of 450 nm was read, and $R_B/R_A$ (the index of R indicates the name of the cured sample) was obtained.

Examples 9, 10, and 14 and Comparative Example 5

Dental curable compositions CR2, 3, 7, and 8 were produced and evaluated in the same manner as those in Example 8 except that the silica composite oxide particulate described in Table 4 was used. The composition and results are shown in Table 4. Note that "↑" in Table means "same as above".

Examples 11 and 13

Under red light, 0.6 parts by mass of CQ, 1.0 part by mass of DMBE, and 0.15 parts by mass of HQME were added to 100 parts by mass of a matrix M2 and mixed to prepare a homogenous polymerizable monomer composition. 200 parts by mass of the silica composite oxide particulate shown in table 4 was weighed out and placed in a mortar, and the polymerizable monomer composition was gradually added thereto under red light and sufficiently kneaded with a pestle to obtain a homogenous curable paste. Further, this paste was degassed under reduced pressure to remove air bubbles to produce dental curable compositions CR4 and CR6. The dental curable compositions CR4 and CR6 were evaluated in the same manner as that in Example 8. The composition and evaluation results are shown in Table 4.

Example 12

Under red light, 0.6 parts by mass of CQ, 1.0 part by mass of DMBE, and 0.15 parts by mass of HQME were added to 100 parts by mass of a matrix M3 and mixed to prepare a homogenous polymerizable monomer composition. 200 parts by mass of the silica composite oxide particulate F5 was weighed out and placed in a mortar, and the polymerizable monomer composition was gradually added thereto under red light and sufficiently kneaded with a pestle to obtain a homogenous curable paste. Further, this paste was degassed under reduced pressure to remove air bubbles to produce a dental curable composition CR5. The dental curable composition CR5 was evaluated in the same manner as that in Example 8. The composition and evaluation results are shown in Table 4.

(Table 4)

| No. | | Dental curable composition | Matrix (part by mass) | Spherical particle (part by mass) | $n_F - n_P$ | $R_B/R_A$ |
|---|---|---|---|---|---|---|
| Example | 8 | CR1 | M1 (100) | F1 (200) | 0.016 | 1.0 |
| | 9 | CR2 | ↑ | F2 (200) | ↑ | 1.1 |
| | 10 | CR3 | ↑ | F3 (200) | ↑ | 1.1 |
| | 11 | CR4 | M2 (100) | F4 (200) | 0.011 | 0.9 |
| | 12 | CR5 | M3 (100) | F5 (200) | 0.015 | 1.0 |
| | 13 | CR6 | M2 (100) | F6 (200) | 0.010 | 1.1 |
| | 14 | CR7 | M1 (100) | F7 (200) | 0.016 | 1.0 |
| Comparative Example 5 | | CR8 | ↑ | F12 (200) | 0.017 | 2.5 |

As shown in Table 4, it can be seen that CR1 to CR7 using the silica composite oxide particulates F1 to F7 that has no silica core structure satisfy the relationship: the ratio $R_B/R_A$ of the spectral reflectance due to the thickness is 0.8 or more and less than 2.0, and the influence of blue scattered light is small. On the other hand, it can be seen that CR8 using the particulate F12 that has a silica core structure has $R_B/R_A$ exceeding 2.0 and exhibits blue scattered light.

3. Examples and Comparative Examples regarding dental blank for cutting (HR blank)

The abbreviations of the polymerization initiator and the pigment used in the preparation of the HR blank are shown below.

· BPO: benzoyl peroxide
· R: red pigment (Pigment Red 166)
· Y: yellow pigment (Pigment Yellow 95)
· B: blue pigment (Pigment Blue 60)

Example 15

After mixing 20 parts by mass of the matrix M1 and 1.0 part by mass of BPO, 80 parts by mass of the silica composite oxide particulate F1 was added thereto and mixed using a planetary mixer until the mixture becomes homogenous, thereby forming a paste. A coloring agent prepared by mixing R: 650 mass ppm, Y: 1800 mass ppm, and B: 1000 mass ppm was added to 100 parts by mass of the paste to prepare a colored paste toned so as to have the A3 shade of the VITA shade guide at the condition of 1 mm thickness. After the colored paste was degassed under vacuum, a columnar cavity having a thickness of 14.5 mm and a substantially rectangular cross section of 14.5 mm × 18 mm of a mold was filled with the colored paste, and the mold was pressurized with nitrogen at 0.4 MPa in a pressure container and allowed to stand in a heating device at 90°C. The colored paste was polymerized and cured by being heated for 15 hours in this state, cooled to room temperature at a cooling rate of 20°C/min, and then removed from the mold, thereby obtaining a colored HR (cured body) having a block shape corresponding to the shape of the cavity.

Plate-shaped test pieces having main planes of 14.5 mm × 18 mm and thicknesses of 1 mm and 3 mm were cut from the colored HR, and the main planes were polished to a glossy finish to obtain test pieces for visual evaluation. The test pieces for visual evaluation and the VITA shade guide "A3" were placed side by side against a black background, and the color tone compatibility was evaluated by visual evaluation in accordance with the following evaluation criteria. The results are shown in Table 5.

<Evaluation criteria>

A: the color tone matches well with the color tone of the VITA shade guide.
B: the color tone is similar to the color tone of the VITA shade guide.
C: the color tone is similar to the color tone of the VITA shade guide, but the compatibility is not good.
D: the color tone does not match with the color tone of the VITA shade guide.

Examples 16 to 21

Colored HR (cured bodies) were produced in the same manner as that in Example 15 except that the matrix and the silica composite oxide particulate used were those shown in table 5, and evaluated in the same manner as that in Example 15. The results are shown in Table 5.

Comparative Example 6

After mixing 20 parts by mass of the matrix M1 and 1.0 part by mass of BPO, 80 parts by mass F12 was added thereto and mixed using a planetary mixer until the mixture becomes homogenous, thereby forming a paste. A coloring agent prepared by mixing R: 850 mass ppm, Y: 2000 mass ppm, and B: 900 mass ppm was added to 100 parts by mass of the paste to prepare a colored paste toned so as to have the A3 shade of the VITA shade guide at the condition of 1 mm thickness. The colored paste was polymerized by the same operation as that in Example 15 to produce a colored HR (cured body) and evaluated in the same manner as that in Example 15. The results are shown in Table 5.

(Table 5)

| No. | Matrix | Silica composite oxide particulate | Color tone compatibility evaluation result | |
|---|---|---|---|---|
| | | | 1 mm thickness | 3 mm thickness |
| Example 15 | M1 | F1 | A | A |
| Example 16 | ↑ | F2 | A | B |
| Example 17 | ↑ | F3 | A | B |
| Example 18 | M2 | F4 | A | A |
| Example 19 | M3 | F5 | A | A |
| Example 20 | M2 | F6 | A | B |
| Example 21 | M1 | F7 | A | A |
| Comparative Example 6 | ↑ | F12 | A | C |

As shown in Table 5, it can be seen that in Examples 15 to 21 satisfying the conditions of the present invention, a favorable color tone compatibility with the VITA shade guide is achieved in both 1 mm thickness and 3 mm thickness. On the other hand, in Comparative Example 6, although a favorable color tone compatibility with the VITA shade guide is achieved in 1 mm thickness, it showed a higher saturation (stronger color) than the VITA shade guide and the color tone compatibility was inferior to those in Examples, in in 3 mm thickness.

**Claims**

1. A dental curable composition, comprising:

a radically polymerizable monomer: 100 parts by mass; and
a silica composite oxide particulate that includes a composite oxide of silicon and a metal including at least titanium or zirconium, has a silica content ratio of 80 to 92 mol% and an average primary particle size of 350 to 600 nm, and is formed of a spherical particle including no silica core inside: 100 to 800 parts by mass.

2. The dental curable composition according to claim 1, wherein
a standard deviation value of the average primary particle size is 1.00 to 1.30.

3. The dental curable composition according to claim 1 or 2, further comprising
a dye.

4. A dental blank for cutting, comprising:
a to-be-cut portion including a cured body of the dental curable composition according to any one of claims 1 to 3.

5. A method of producing a silica composite oxide particulate, comprising:

a step of preparing a raw material solution by dissolving a condensable silicon compound and a condensable metal compound in a first solvent including a first organic solvent; and
a step of precipitating, by adding the raw material solution to a basic solution including a second solvent that includes a second organic solvent and water to hydrolyze and condense the condensable silicon compound and the condensable metal compound, a silica composite oxide particulate that includes a composite oxide of silicon and a metal including at least titanium or zirconium, has a silica content ratio of 80 to 92 mol%, and is formed of a spherical particle,
the method of producing a silica composite oxide particulate being **characterized in that**
in the first organic solvent, a content ratio of methanol is 80 mass% or more,
in the second organic solvent, a total content ratio of an alcohol having an alkyl group that has 3 carbon atoms and an alcohol having an alkyl group that has 4 to 5 carbon atoms is 80 mass% or more and a content ratio of an alcohol having an alkyl group that has 3 or less carbon atoms is 5 to 50 mass%, and
the silica composite oxide particulate that has an average primary particle size of 350 to 600 nm and includes no silica core inside the spherical particle is precipitated by adding the raw material solution to the basic solution such that a content ratio of an alcohol having an alkyl group that has 3 to 5 carbon atoms is 65 mass% or more with respect to a total mass of the first organic solvent and the second organic solvent.

6. The method of producing a silica composite oxide particulate according to claim 5, wherein
the step of preparing a raw material solution includes

a step of preparing, by mixing a silicon alkoxide, methanol, water, and an acid, a first composition in which a partial hydrolysate of the silicon alkoxide and/or an oligomer obtained by condensing the partial hydrolysate is dissolved,
a step of preparing, by mixing a metal alkoxide of the metal and a polar organic solvent for dissolving the metal alkoxide, a second composition in which at least one of the metal alkoxide, a partial hydrolysate of the metal alkoxide, or an oligomer obtained by condensing the partial hydrolysate is dissolved, and
a step of preparing the raw material solution by mixing the first composition, and the second composition.

7. The method of producing a silica composite oxide particulate according to claim 5 or 6, wherein
a standard deviation value of the average primary particle size is within a range of 1.00 to 1.30.

8. A dental filler, which is blended in a dental curable composition that includes a radically polymerizable monomer, **characterized in that**,
the dental filler includes a composite oxide of silicon and a metal including at least titanium or zirconium, has a silica content ratio of 80 to 92 mol% and an average primary particle size of 350 to 600 nm, and is a silica composite oxide particulate formed of a spherical particle including no silica core inside, a standard deviation value of the average primary particle size being with a range of 1.00 to 1.30.

9. The dental filler according to claim 8, wherein

the radically polymerizable monomer is a (meth)acrylic compound radically polymerizable monomer, and
the dental filler satisfies the following formula: $0.01 < n_F - n_P < 0.1$, wherein
$n_F$ represents a refractive index of the spherical particle at 25°C and with respect to light of a wavelength of 589 nm and $n_P$ represents a refractive index of a cured body of the radically polymerizable monomer at 25°C and with respect to light of a wavelength of 589 nm.

**Amended claims under Art. 19.1 PCT**

1. A method of producing a silica composite oxide particulate, comprising:

a step of preparing a raw material solution by dissolving a condensable silicon compound and a condensable metal compound in a first solvent including a first organic solvent; and
a step of precipitating, by adding the raw material solution to a basic solution including a second solvent that includes a second organic solvent and water to hydrolyze and condense the condensable silicon compound and the condensable metal compound, a silica composite oxide particulate that includes a composite oxide of silicon and a metal including at least titanium or zirconium, has a silica content ratio of 80 to 92 mol%, and is formed of a spherical particle,
the method of producing a silica composite oxide particulate being **characterized in that**
in the first organic solvent, a content ratio of methanol is 80 mass% or more,
in the second organic solvent, a total content ratio of an alcohol having an alkyl group that has 3 carbon atoms and an alcohol having an alkyl group that has 4 to 5 carbon atoms is 80 mass% or more and a content ratio of an alcohol having an alkyl group that has 3 or less carbon atoms is 5 to 50 mass%, and
the silica composite oxide particulate that has an average primary particle size of 350 to 600 nm and includes no silica core inside the spherical particle is precipitated by adding the raw material solution to the basic solution such that a content ratio of an alcohol having an alkyl group that has 3 to 5 carbon atoms is 65 mass% or more with respect to a total mass of the first organic solvent and the second organic solvent.

2. The method of producing a silica composite oxide particulate according to claim 1, wherein
the step of preparing a raw material solution includes

a step of preparing, by mixing a silicon alkoxide, methanol, water, and an acid, a first composition in which a partial hydrolysate of the silicon alkoxide and/or an oligomer obtained by condensing the partial hydrolysate is dissolved,
a step of preparing, by mixing a metal alkoxide of the metal and a polar organic solvent for dissolving the metal alkoxide, a second composition in which at least one of the metal alkoxide, a partial hydrolysate of the metal alkoxide, or an oligomer obtained by condensing the partial hydrolysate is dissolved, and
a step of preparing the raw material solution by mixing the first composition, and the second composition.

3. The method of producing a silica composite oxide particulate according to claim 1 or 2, wherein
a standard deviation value of the average primary particle size is within a range of 1.00 to 1.30.

4. A dental filler, which is blended in a dental curable composition that includes a radically polymerizable monomer, **characterized in that**,
the dental filler includes a composite oxide of silicon and a metal including at least zirconium, has a silica content ratio of 80 to 92 mol% and an average primary particle size of 350 to 600 nm, and is a silica composite oxide particulate formed of a spherical particle including no silica core inside, a standard deviation value of the average primary particle size being with a range of 1.00 to 1.30.

5. The dental filler according to claim 4, wherein

the radically polymerizable monomer is a (meth)acrylic compound radically polymerizable monomer, and
the dental filler satisfies the following formula: $0.01 < n_F - n_P < 0.1$, wherein
$n_F$ represents a refractive index of the spherical particle at 25°C and with respect to light of a wavelength of 589 nm and $n_P$ represents a refractive index of a cured body of the radically polymerizable monomer at 25°C and with respect to light of a wavelength of 589 nm.

6. A dental curable composition, comprising:

a radically polymerizable monomer: 100 parts by mass; and
a silica composite oxide particulate that includes a composite oxide of silicon and a metal including at least zirconium, has a silica content ratio of 80 to 92 mol% and an average primary particle size of 350 to 600 nm, and is formed of a spherical particle including no silica core inside: 100 to 800 parts by mass.

7. The dental curable composition according to claim 6, wherein
a standard deviation value of the average primary particle size is 1.00 to 1.30.

8. The dental curable composition according to claim 6 or 7, further comprising a dye.

9. A dental blank for cutting, comprising:
   a to-be-cut portion including a cured body of the dental curable composition according to any one of claims 6 to 8.

## INTERNATIONAL SEARCH REPORT

<table>
<tr><td></td><td>International application No.<br><br>**PCT/JP2023/029044**</td></tr>
</table>

| A. | CLASSIFICATION OF SUBJECT MATTER |
|---|---|

*A61K 6/849*(2020.01)i; *A61C 5/60*(2017.01)i; *A61C 13/00*(2006.01)i; *A61C 13/15*(2006.01)i; *A61K 6/878*(2020.01)i; *A61K 6/887*(2020.01)i; *C01B 33/18*(2006.01)i

FI:    A61K6/849; A61K6/887; A61K6/878; C01B33/18 Z; A61C5/60; A61C13/15; A61C13/00 A

According to International Patent Classification (IPC) or to both national classification and IPC

| B. | FIELDS SEARCHED |
|---|---|

Minimum documentation searched (classification system followed by classification symbols)

A61K6/849; A61C5/60; A61C13/00; A61C13/15; A61K6/878; A61K6/887; C01B33/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| X | JP 58-110414 A (TOKUYAMA SODA KK) 01 July 1983 (1983-07-01)<br>claims, p. 5, upper left column, line 1 to p. 6, lower left column, line 7, p. 7, upper right column, line 15 to p. 10, upper left column, line 9, p. 10, lower left column, line 4 to p. 11, lower left column, line 2, production examples 23, 90 | 1-4, 8-9 |
| A | | 5-7 |
| X | JP 1-38044 B2 (TOKUYAMA SODA KK) 10 August 1989 (1989-08-10)<br>claims, column 6, line 19 to column 16, line 29, column 17, line 33 to column 18, line 34, production examples 51, 52 | 1-4, 8-9 |
| A | | 5-7 |
| A | JP 2012-153640 A (TOKUYAMA DENTAL CORP) 16 August 2012 (2012-08-16)<br>entire text | 1-9 |
| A | JP 62-86003 A (TOKUYAMA SODA KK) 20 April 1987 (1987-04-20)<br>entire text | 1-9 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 October 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2023/029044** |

### C.    DOCUMENTS CONSIDERED TO BE RELEVANT

| Category* | Citation of document, with indication, where appropriate, of the relevant passages | Relevant to claim No. |
| --- | --- | --- |
| A | JP 8-12305 A (TOKUYAMA CORP) 16 January 1996 (1996-01-16)<br>entire text | 1-9 |

Form PCT/ISA/210 (second sheet) (January 2015)

## INTERNATIONAL SEARCH REPORT
### Information on patent family members

International application No.

**PCT/JP2023/029044**

| Patent document cited in search report | | | Publication date (day/month/year) | Patent family member(s) | Publication date (day/month/year) |
|---|---|---|---|---|---|
| JP | 58-110414 | A | 01 July 1983 | US 4567030 A claims, column 3, line 22 to column 11, line 57, column 12, line 61, column 13, line 34 | |
| JP | 1-38044 | B2 | 10 August 1989 | (Family: none) | |
| JP | 2012-153640 | A | 16 August 2012 | (Family: none) | |
| JP | 62-86003 | A | 20 April 1987 | (Family: none) | |
| JP | 8-12305 | A | 16 January 1996 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3010603 B
- JP 8012305 A
- JP 1038044 B
- JP 2017213394 A